# EUROPEAN PATENT SPECIFICATION

(11) **EP 2 749 200 B1**
(45) Date of publication and mention of the grant of the patent: **27.02.2019**
(21) Application number: 13199314.9
(22) Date of filing: 23.12.2013
(51) Int. Cl.: A61B 1/00, A61B 1/045, G06F 9/445, H04N 5/232

(54) **Reprogrammable video imaging system with modular architecture**
Wiederprogrammierbares Videobilderzeugungssystem mit modularer Bauweise
Système d'imagerie vidéo reprogrammable à architecture modulaire

(30) Priority: 31.12.2012 US 201213731155
(43) Date of publication of application: 02.07.2014
(73) Proprietor: Karl Storz Imaging, Inc., Goleta, CA 93117 (US)
(72) Inventor: Amling, Marc R., Goleta, CA 93117 (US); Aiken, Scott, Goleta, CA 93117 (US)
(74) Representative: Witte, Weller & Partner Patentanwälte mbB

(56) References cited:
- US-A- 5 740 801
- US-A1- 2006 055 793
- US-A1- 2007 024 717
- US-A1- 2008 116 093
- US-A1- 2008 198 223
- US-A1- 2010 238 278
- US-A1- 2011 164 126

## Description

### FIELD OF THE INVENTION

The invention relates to a modular camera control device for processing video signals from a variety of camera types and, more particularly, the invention relates to the reprogramming of the modular camera control device.

### BACKGROUND OF THE INVENTION

The field of endoscopy, to which the present invention relates, includes medical diagnostic and therapeutic disciplines that utilize endoscopes to view otherwise inaccessible body cavities using minimally invasive surgical procedures. Endoscopic cameras are typically small and lightweight for ease of use by medical professionals. Typically, the camera is connected to a Camera Control Unit ("CCU"), with the CCU processing and displaying the imaging data from the camera. An example of such an endoscopic modular imaging device is disclosed in US 2011/0164126. Often, each medical procedure requires a different camera, leading to a large inventory of cameras. Additionally, each camera must be compatible with the CCU to function correctly. Each CCU has software to process and operate a variety of camera technologies, and as new technologies become available, the CCU may need updated software to properly process images from new camera technology. Additionally, the CCU hardware may become outdated, thus requiring an entirely new CCU to process the images both old and new camera technologies used by a physician.

CCUs can be designed with robust reprogramability and reconfigureability capabilities, this way, an older model CCU can be upgraded or configured to work with new camera technology. However, rather than reprogramming or reconfiguring a CCU, it is often less costly to replace the older module CCU with a new one because the configuration management of the CCU is often a time and labor intensive process that is difficult to manage efficiently. Furthermore, as software feature business models become prevalent, it becomes more and more difficult to manage which customers purchased what software features.

In known systems, cameras, such as charge coupled devices and the like, used during endoscopic surgery are typically referred to as heads or camera heads. To achieve the desired size and weight of the camera heads, camera head and/or integrated endoscope-camera assembly electronics are typically separated physically from the majority of circuitry required to process and output high-quality, color video images. Typically, to support the miniaturization of camera head image acquisition components, the majority of electronic hardware is housed in the CCU. In known systems, CCUs may be placed on or in carts, or may be permanently wall mounted.

Although current CCU devices allow for upgradeability, each new camera head may include software required to update a CCU to be compatible with that (or an identical) camera head. Since many procedures require different cameras, the CCU must be properly maintained and updated to be compatible with each camera. Therefore, it is important to have an efficient way to manage software updating and reprogramming of camera heads and/or imaging devices.

When image data is, acquired, or picked up, it is sent by the camera head to the CCU. Upon receiving the image data from the camera head, the CCU normally processes the signal to display the acquired image on a viewing device. Generally, the image is used by a medical professional and/or for storage on various media (video cassette recorder, floppy disk, hard drives, flash drives, compact disks, digital video disks, and the like) and/or for transmission to remote locations in various manners, such as by the Intranet, Internet, radio transmission, and the like.

Additionally, the CCU may send commands to the camera head to adjust various settings (i.e. color balance, electronic shutter for light sensitivity, and other optical and electronic characteristics).

Traditionally, CCUs are compatible with a limited number of camera heads. A CCU's hardware is usually difficult to configure for proper communication with varying types of camera heads because camera heads use varying types of imaging devices that can differ in pixel resolution, timing requirements (i.e. PAL, NTSC, Progressive, and other formats), signal output type (i.e. analog or digital), physical size, and in other characteristics.

Digital video systems are typically differentiated by their application. Advanced television (ATV), high definition television (HDTV), and computer systems may differ in format and signal characteristics. In some areas, digital video formats and standards are currently being developed and adopted. The Society of Motion Picture and Television Engineers (SMPTE) is typically in the business of defining and adopting voluminous digital video formal standards. As each is adopted, various applications, and application improvements generally will also be realized. Some examples of digital video encoding/decoding standards and interfaces currently in use are:High-Definition Multimedia Interface (HDMI), Digital Visual Interface (DVI), Serial Digital Interface (SDI), DisplayPort (VESA), Digital Component Video (ITU-R BT.601), Unified Display Interface (UDI), FireWire (IEEE-1394), and Universal Serial Bus (USB).

Furthermore, there may be variability from device to device of the same type, which may affect camera head performance. For example, camera heads may differ in scanning principles, resolution capability, sampling rates, aspect ratios, synchronization, bandwidth, and the like. Additionally, commands sent from the CCU to the camera head are generally unique depending upon the camera head type being used. Moreover, as repairs, modifications, or improvements are made to camera heads, the CCU, which was originally designed to be compatible with the older camera head, may become incompatible and may require upgrading as well.

This overall variability in camera heads, either caused by imaging device technologies or by CCU command characteristics, often results in a CCU being specifically designed to be compatible with the camera head type being utilized. Also, consumers may desire different capabilities related to specific applications of the cameras, such as for medical, industrial, and scientific uses. Capabilities include picture in picture (PIP), reverse video (image flip), electronic zoom, still image capture, and stereoscopic video interface.

Moreover, CCUs are typically designed for use with camera head technologies currently in existence, and not designed to anticipate and accommodate camera heads yet to be developed. Hence, CCUs are typically not designed to be compatible with future camera head technologies; particularly, image device and image signal transmission technologies. These differences between older and newer camera heads also contribute to compatibility problems.

Because CCUs are usually compatible with limited quantities of camera heads, CCUs are typically discarded in favor of ones that were designed concurrently and/or to be compatible with particular camera head technologies. Consequently, CCUs have become an added expense often associated with changing imaging devices or camera heads. Further, it is typically desired for camera heads to be improved due to the demand from consumers to have the latest technology and advancement in equipment. Moreover, CCUs used in medical and veterinary fields are increasingly being mounted permanently in equipment bays or carts and/or permanently mounted within the walls of surgical operating rooms themselves. The expense associated with replacing CCUs to maintain compatibility with camera heads is subsequently passed onto consumers.

Thus there exists a need for a modular system that overcomes the disadvantages of the prior art. It is further desired that the camera head or CCU may be updated or reprogrammed in an efficient and cost effective manner, rather than replacing the older camera head or CCU with a newer module. It is yet further desirable to provide a modular system, including camera heads and CCUs, that is readily compatible with existing and future imaging technologies and that allows for camera heads and CCUs to be backwards and forwards compatible.

### SUMMARY OF THE INVENTION

Accordingly, it is an object of the present is to provide a camera control device having a modular architecture capable of being updated.

Another object of the present invention is to provide a modular camera control device capable of processing and displaying data from a variety of camera types and imaging sources both existing and developed in the future.

Another object of the present invention is to provide a modular camera control device that can be updated in an efficient manner.

Another object of the present invention is to reduce the total cost of ownership for a visualization system.

Another object of the present invention is to increase the likelihood that a newly purchased visualization system will work with existing equipment.

Another object of the present invention is to provide a modular camera control device that may be efficiently updated for expanded software or hardware functionality purchased by the consumer.

Yet another object of the present invention is to provide a modular camera control device that may be updated in field.

These and other objects of the invention are achieved by providing a reprogrammable modular imaging device having a control module and one or more input modules, each of the modules having a processor and being configured to being reprogrammed including a compatibility check as defined by claim 1.

The reprogrammable modular imaging device has a control module with a processor and one or more input modules with a processor. The input modules are connectable to the control module. Image data is received by the least one input module for processing and transmission to the control module. Processed image data is received by the control module for display formatting, and a program is received by the control module to reprogram the processor of the control module and the processor of the input module.

The image data is raw image data, and the input module may transmit processed image data to the control module in a format readable by the control module. The processed image data can be in a format readable by the control module.

A camera is connectable to the input module for transmitting image data, and a display can be connected to the control module for displaying processed and formatted image data. The camera can have a processor and a program may be received by the control module to reprogram the processor of the camera. The program can be received by the control module through an upgrade port. The input module may receive the program from the control module. The reprograming of the processor may reconfigure the processor, enable a soft feature and disable a soft feature. A module link connecting the control module to the input module allows the input module to receive the program from the control module. Data and commands may be transmitted through the module link.

The program received by the control module may come through a network connection. The program may also be retrieved by the control module through a network connection. The network connection may optionally be wireless. The program may also reprogram the modular imaging device to be compatible with an alternate image source or alternate image module. Further, processor can receive an authorization for the reprogram.

### BRIEF DESCRIPTION OF THE DRAWINGS

FIGS. 1A and 1B are respectively, front and rear perspective views of an embodiment of the invention.
FIG. 2 is a schematic of the invention shown in Figures 1A and 1B.
FIG. 3 is a flow chart showing how the compatibility of the program of Figure 1A is determined.
FIGS. 4A and 4B are flow charts showing how the program of Figure 1A is authorized.
FIG. 5 is a flow chart showing how the control module of Figures 1A and 1B is reprogrammed.
FIG. 6 is a flow chart showing how an input module of Figures 1A and 1B is reprogrammed.
FIG. 7 is a flow chart showing how the program of Figure 1A is requested from an external storage location.

### DETAILED DESCRIPTION OF THE INVENTION

A modular architecture of the camera control device allows the consumer increased flexibility. The modular camera control device allows upgradeability and compatibility with a multitude of camera heads that are supported by a plurality of input modules, the camera heads and input modules may be existing or yet to be developed. Formerly, when a new imaging technology became available, a camera control unit could be incompatible with the new technology due to a variety of constraints, for example, hardware.

By using a modular architecture, the new technology can be supported by an input module that is compatible with the control module. In order to streamline the flexibility of the modular architecture, it is important to have an efficient way to re-program or re-configure the modular camera control device software, firmware, drivers etc. Further, because the program is loaded onto the control module, there is no need for the user to follow a particular set of steps to configure both old and new modules to work together. Instead, the user loads a program or a compressed file having a number of programs onto the control module. The program is installed for each module that needs updating without the need to separately, boot, install, configure, program, re-boot, etc., each module. The compressed file with a number of programs contains all files necessary for updating the modular unit, and data links between the control module and each input module allow for reprogramming as necessary.

A compatibility check can be done for software and hardware with limited interaction from the user. The modular architecture increases the likelihood that existing visualization technology and yet to be developed visualization will be able to operate with some if not all of the same image processing hardware. This results in decreased capital costs for physicians and medical offices.

The control module is designed to accommodate general image processing and display functions for multiple camera types or families. These general functions include, for example, user interface, image capture and streaming functionality as well as input/output functionality for the display/monitor interfaces, system interface and control, and network connectivity. The control module can be designed to accommodate one or multiple imaging modules.

In the example of a control module that supports only one input module at a time, the overall modular device can be purchased at a lower initial cost. If the consumer wishes to purchase different camera or input module types, the modular device may be re-programmed to work with different imaging technology. If the control module supports multiple input modules, the consumer may still purchase new imaging technology, cameras and/or input modules, and still use the same control module once the reprogramming is completed.

The input modules support all functions required for a group or family of image sources, such as cameras or auxiliary inputs. The input module provides compatibility between the family of image sources and the control module. Over the life of the system, additional input modules may be purchased to support emerging imaging technology such as 3D imaging, advanced fluorescence imaging, solid-state variable direction of view endoscopes, wireless camera heads and the like.

The group of input modules connected to the control module may include an auxiliary input module. This module supports a variety of video sources such as third party camera control units, C-Arm, X-Ray, Ultrasound, Personal Computers, endoscopic camera systems, room cameras, and the like. Supported input formats may include, DVI, VGA, S-Video, Composite, 3G-SDI and the like. Inputs may be both automatically and manually selected. The auxiliary module provides increased backward compatibility, forward compatibility and third party image source compatibility.

The re-programmability function of the modular architecture allows for economical buyers to progressively upgrade their imaging technology, rather than being required to purchase a camera control unit that is compatible with the entire range of imagers that the buyer would wish to purchase in the future. The efficient re-programmability function allows for hardware upgrades, reconfiguration and software feature upgrades. The re-programmability function further minimizes the likelihood that newly purchased visualization technology will become obsolete while increasing backward compatibility of upgrades. Further, the cost of ownership and upgrade, such as acquisition, back-up, and maintenance, is reduced.

The re-programmability of the modular device further allows software features to be selectively activated in a cost effective manner. Since the reprogramming step is relatively simple compared to the prior art, it is easier to sell software features to the end user. The modular device can retrieve or receive a program through a LAN connection, allowing the manufacturer or software provider to reprogram the system remotely. Further, the modular camera control device may be connected to an Internet/Ethernet connection, allowing updates to be purchased, downloaded or verified online, with the files sent to the control device through the data connection.

By loading a program on the processor of the input module and/or the control module, a number of features can be modified. The reprogram can reconfigure and/or reprogram: hardware, drivers, input/output interfaces, user interfaces, display features, camera features, camera processors, color and white balance functions, and software features. The reprogram can also modify the modular imaging device to be compatible with alternate input modules and alternate image sources such as a new camera type.

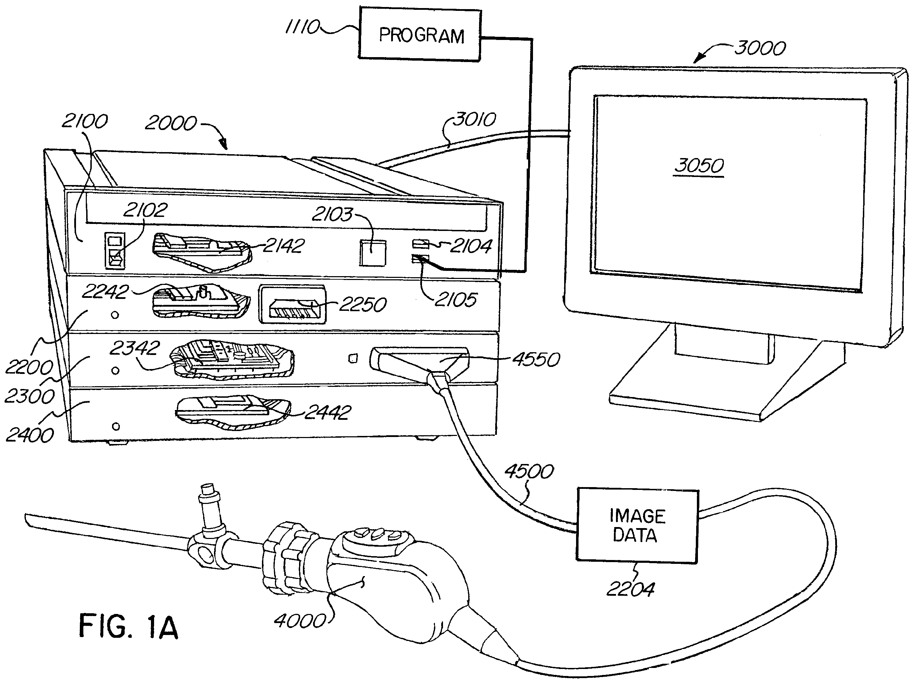

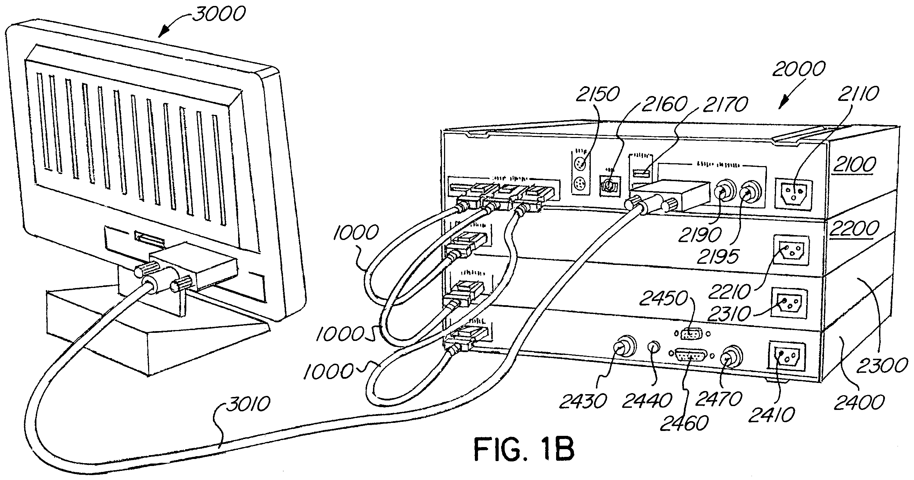

FIGS. 1A and 1B show a control module 2100 connected to one or more input modules 2200. Each input module is connected to the control module with a data link 1000. The control module receives a program 1110. In the present example, the program 1110 is received through a port 2105, however the program 1110 may be received through other port or data connection types as would be apparent to one of skill in the art. The program reprograms one or multiple processors 2142, 2242, 2342, 2442.

The control module 2100 is responsible for display formatting processed image data received from the input module 2200, 2300, 2400. The control module may perform a number of commands and functions such as: zoom, image orientation, image rotation, image enhancement, 2D to 3D conversion, 3D to 2D conversion, picture-in-picture, graphical user interface overlay, and the like. The data link 1000 receives and sends processed image data, commands, software updates and other data between the control module 2100 and the input module 2200, 2300, 2400..

Each control module 2100 and input modules 2200, 2300 and 2400 have a power plug 2110, 2210, 2310 and 2410 respectively. Control module 2100 may have a plurality of slots, for example, slots 2110, 2120, 2130, 2140 for receiving the cable 1000. Each of slots 2110, 2120, 2130 and 2140 can be connected to a separate input module.

If an auxiliary input module 2400 is connected, the program can also reprogram the auxiliary input module processor 2442. The auxiliary module 2400 may allow one or more auxiliary sources 2430, 2440, 2450, 2460 and 2470 to connect to various other input and output devices. Examples of auxiliary sources may include VGA, S-Video, DVI, SDI, HD-SDI, and 3G-SDI sources, a camera control unit, an operating room camera, a computer, or other data sources as desired.

The camera 4000 and the input module 2300 are connected with a link 4500. As shown in the Figures, the link is a cable, however other data transmission devices such as wireless, optical or other can be used as would be apparent to one of skill in the art. Control module 2100 also has various output and input elements 2150, 2160, 2170, 2180, 2190 and 2195 to connect to various other input and output devices. Example input/output elements may include DVI output for a DVI monitor or recorder, and a 3G SDI output for 3G SDI monitors or recorders. As shown, the control module is connected to a display 3050 with a cable 3010.

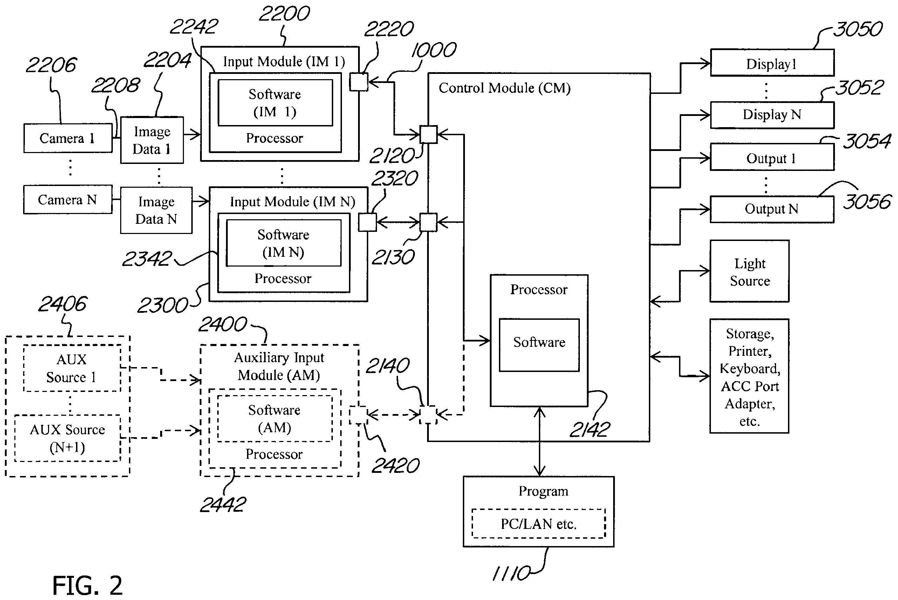

FIG. 2 shows an input module 2200 designed to process image data 2204 from a camera 2206. Each input module processes data from a camera family or type. Additional input modules can be added to the system to accommodate different camera types or families. The input module processes image data 2204 from a camera 2206 and sends the processed data to the control module 2100 through the data link 1000.

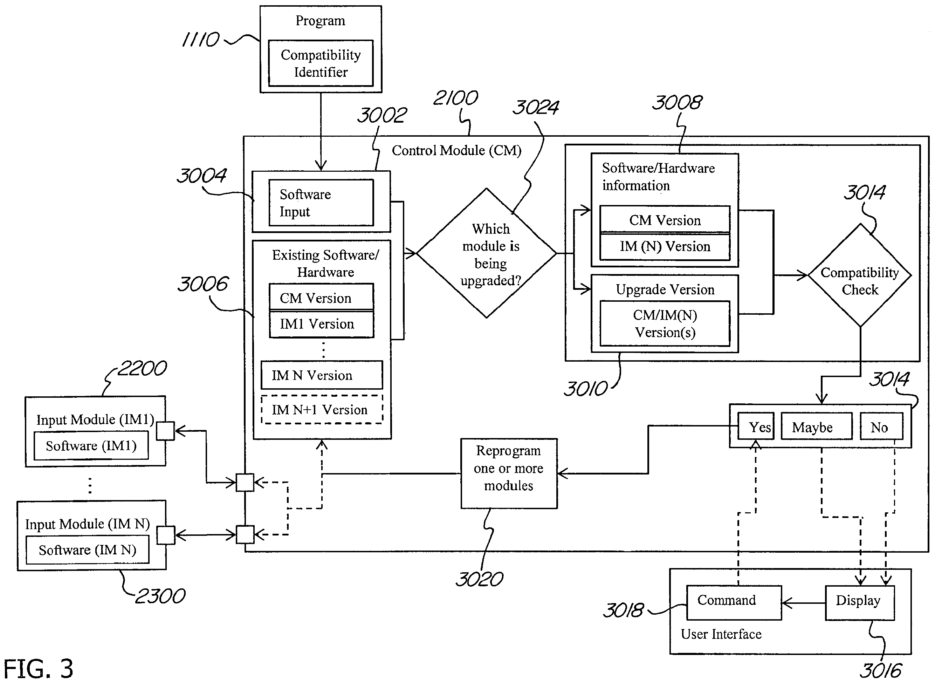

FIG. 3 shows program 1110 loaded onto the control module 2100 through an optional data port 1120. The program 1110 may contain a collection of files such as drivers, firmware or other data used for programming or reprogramming the one or more input modules 2200, 2300, 2400 and/or the control module 2100. The data port type used will depend on how the program is loaded. For example, if the program is loaded through a USB flash drive, the data port used would be one of the USB ports shown in previous figures. If the program is loaded through Internet/Ethernet connections, the data port used could be an Internet/Ethernet port. Once the user initiates the programming or reprogramming of the imaging device, it must be determined which module(s) will be updated. A version comparison 3024 is done to determine the software input version 3004 with the existing versions 3006 of the input or control modules currently installed. Once it is confirmed which module will be updated, a compatibility check 3012 compares the current software/hardware 3008 with the inputted software 3010 to determine if the update will be compatible. If the result 3014 of the compatibility check 3012 is "Yes," the software is upgraded for each module that the upgrade pertains to. If the result 3014 of the compatibility check 3012 is "Maybe," the control module 2100 displays 3016 the result 3014 of the compatibility check 3012 so that the user may initiate a command 3018 to upgrade the module 3020 despite the "Maybe," result 3014. After the compatibility determination and result, the existing software 3006 on the control module 2100 or the software on one or more input modules 2200 is updated according to the compatibility result 3014. When an input module is updated, the file pertaining to each input module from the program 1110 is sent to the input module 2200 through a data link 1000 that connects the control module 2100 to the input modules 2200, 2300 etc.

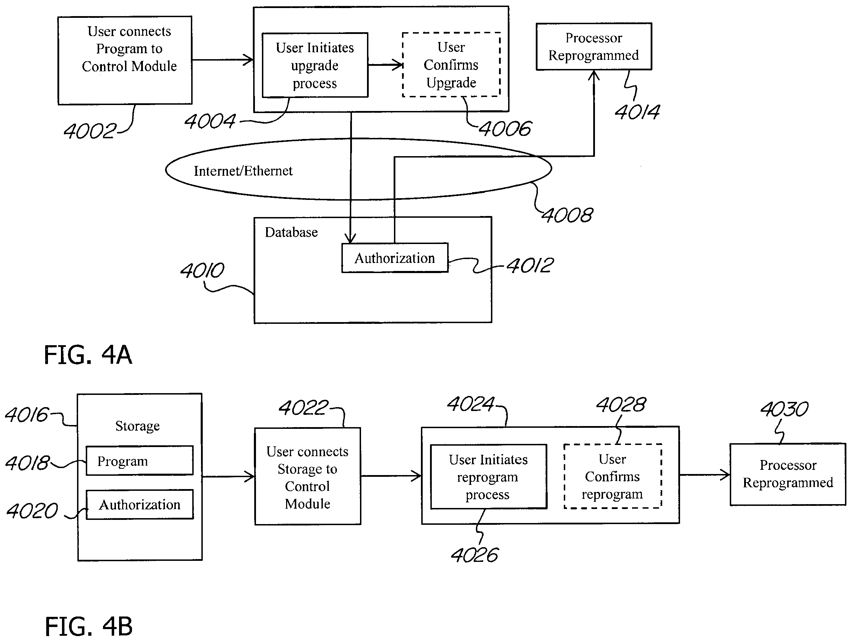

FIG. 4A shows a program connected to the control module 4002 and the user initiates the upgrade process 4004. To initiate the upgrade, the user may enter a command 4006 that confirms the upgrade. Once the upgrade is initiated 4002 or confirmed 4006, the request is transmitted over the Internet/Ethernet 4008 to a database 4010 containing an authorization code 4012. Assuming the device is authorized to be upgraded, the Authorization code 4012 is sent back to the control module, allowing the software to be installed 4014 on the modular imaging device.

FIG. 4B an external storage 4016 connected 4022 to the control module. The storage 4016 contains the program 4018 and the authorization code 4020. The upgrade process 4026 is initiated and the software with authorization code is sent to the control module. The user optionally confirms 4028 the upgrade. Once the upgrade is confirmed, the updated software 4030 is installed on the modular imaging device.

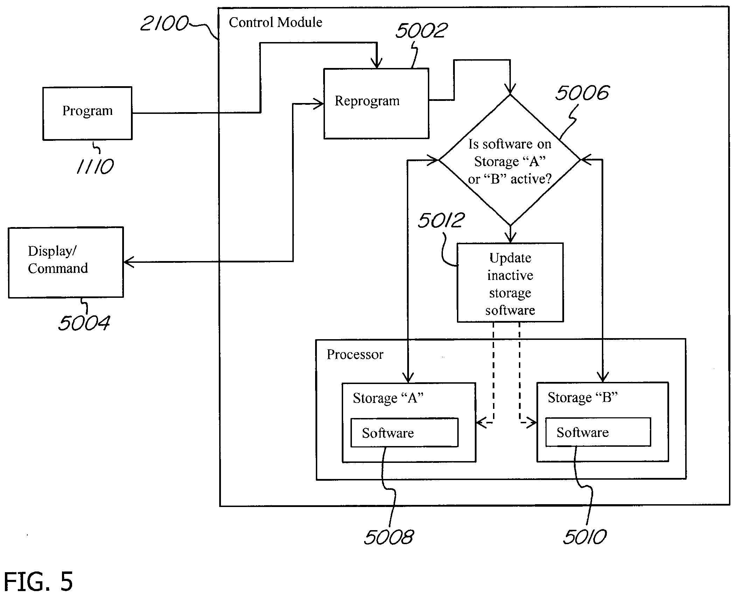

FIG. 5 shows a program 1110 loaded through a data port and the reprogramming process 5002 begins. A confirmation that the reprogramming will begin can be displayed 5004 and the user can send back a positive command. The system may check the compatibility and eligibility 5014 of hardware. It may be necessary to re-program the control module while still powered on, which requires the active software to re-program an alternate storage and then later switch to the alternate storage. The system determines which storage is active 5006. Once the active storage is confirmed, the inactive storage is updated 5012 with the new software. For example, if the software on storage "A" is active, the new software is loaded onto storage "B" and then the module switches to using the software on storage "B" as the active software.

In addition, a program can be loaded onto the control module and the program 6002 is transmitted to the input module 2200. Once the control module sends commands and program(s) to the input module 6002, reprogramming process begins. The system determines which storage is active 6004. Once the active storage is confirmed, the inactive storage is updated 6006 with the new software. For example, if the software on storage "A" 6008 is active, the new software is loaded onto storage "B" and then the module switches to using the software on storage "B" 6010 as the active software. The result or status of the update is determined 6012 and the input module confirms the status of the upgrade with the control module 6014.

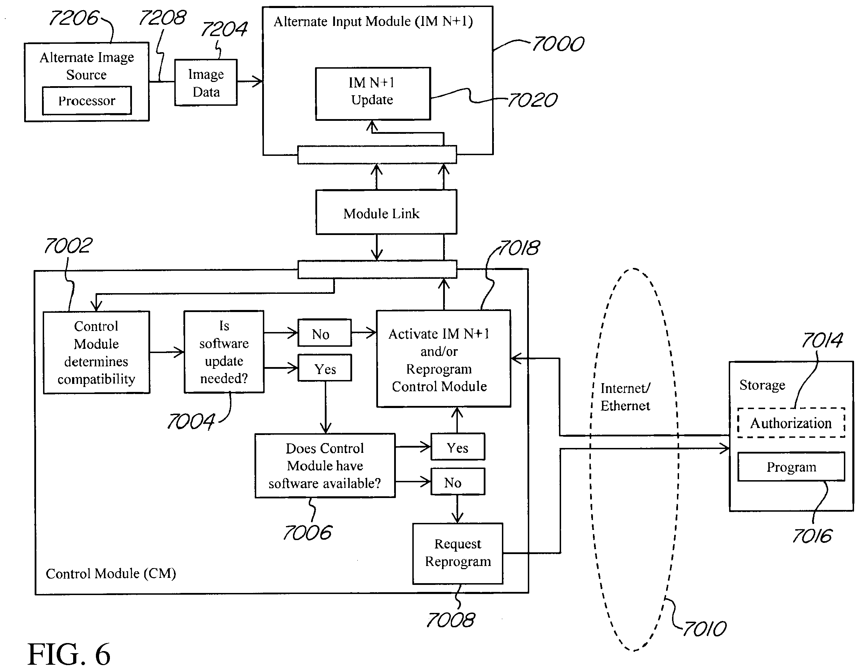

FIG. 6 shows an alternate input module 7000 connected to the control module 2100. The alternate input module may be a newly purchased module, or a module that is used for a particular family of cameras or medical procedure. The control module determines compatability 7002 of the alternate module 7000 and the control module 2100. If a software update is not needed, the alternate module 7000 is activated. If a software update is needed, and the software is already available, the control module may be reprogrammed 7018 as necessary. If the alternate input module requires reprogramming, the control module updates the alternate input module 7020. If the update for the control 2100 or alternate 7000 module is not available, the control module may request the reprogram 7008. The figure shows a request to an external storage 7012. The request may be made by displaying an update request to a user (not shown) or by a request over an internet connection 7010. The external storage sends the program 7016 to the control module 2100 and optionally includes an authorization 7014. Once the control module 2100 has the necessary program, the activation/re-program 7018 can occur for the control module and the control module can update the input module 7020 as necessary.

## Claims

1. Reprogrammable modular imaging device (2000) comprising:
a control module (2100) having a processor (2142);
at least one input module (2200; 2300; 2400) having a processor (2242; 2342; 2442) and connectable to said control module (2100);
a camera (2206; 4000) connectable to the at least one input module (2200; 2300; 2400) for transmitting raw image data (2204) to the at least one input module (2200; 2300; 2400);
said at least one input module (2200; 2300; 2400) processing said raw image data (2204) to generate processed image data for transmission to said control module (2100);
said control module (2100) receiving said processed image data and formatting said processed image data for display; and **characterised in that** said modular imaging device (2000) further comprises:
a program (1100) received by said control module (2100) as inputted software (3010) to reprogram both the processor (2242; 2342; 2442) of said at least one input module (2200; 2300; 2400) and the processor (2142) of said control module (2100),
wherein a compatibility check (3012) compares a current software/hardware (3008) of the control module (2100) with the inputted software (3010) to determine if the inputted software (3010) will be compatible, and if the result (3014) of the compatibility check is "Yes", at least one module (3020) is reprogrammed to which the inputted software (3010) pertains to, and if the result (3014) of the compatibility check (3012) is "Maybe", the control module (2100) displays (3016) the result (3014) of the compatibility check (3012) so that the user may initiate a command (3018) to reprogram the module (3020) despite the "Maybe" result (3014).

2. Device of claim 1, wherein said control module (2100) has an upgrade port (2105) for receiving the program (1100).

3. Device of any preceding claim, wherein the at least one input module (2200; 2300; 2400) receives the program (1100) from the control module (2100).

4. Device of any preceding claim, wherein said reprogram reconfigures both the processor (2142; 2242; 2342; 2442) of said at least one input module (2200; 2300; 2400) and the processor (2142) of said control module (2100).

5. Device of any preceding claim further comprising a soft feature enabled by the reprogram.

6. Device of any preceding claim further comprising a soft feature disabled by the reprogram.

7. Device of any preceding claim further comprising a module link (1000) connecting the control module (2100) to the at least one input module (2200; 2300; 2400).

8. Device of any preceding claim further comprising a network connection (7010), the program (1100) received from said network connection (7010).

9. Device of claim 8 wherein the network connection (7010) is wireless.

10. Device of any preceding claim further comprising an alternate image source (7206) compatible with the processor (2142) upon the reprogram.

11. Device of any preceding claim further comprising a reprogram authorization (7014) received by the processor (2142).

12. Device of any preceding claim further comprising a program received by the control module (2100) to reprogram a processor of the camera (2206; 4000).

## Patentansprüche

1. Umprogrammierbare modulare Bildaufnahmevorrichtung (2000), aufweisend:
ein Steuermodul (2100) mit einem Prozessor (2142);
mindestens ein Eingangsmodul (2200; 2300; 2400) mit einem Prozessor (2242; 2342; 2442), das mit dem Steuermodul (2100) verbindbar ist;
eine Kamera (2206; 4000), die mit dem mindestens einen Eingangsmodul (2200; 2300; 2400) verbindbar ist, um Rohbilddaten (2204) an das mindestens eine Eingangsmodul (2200; 2300; 2400) zu übertragen;
wobei das mindestens eine Eingangsmodul (2200; 2300; 2400) die Rohbilddaten (2204) verarbeitet, um verarbeitete Bilddaten zur Übertragung an das Steuermodul (2100) zu erzeugen;
wobei das Steuermodul (2100) die verarbeiteten Bilddaten empfängt und die verarbeiteten Bilddaten zur Anzeige formatiert; und **dadurch gekennzeichnet, dass** die modulare Bildaufnahmevorrichtung (2000) ferner aufweist:
ein Programm (1100), das von dem Steuermodul (2100) als eingegebene Software (3010) empfangen wird, um sowohl den Prozessor (2242; 2342; 2442) des mindestens einen Eingangsmoduls (2200; 2300; 2400) als auch den Prozessor (2142) des Steuermoduls (2100) neu zu programmieren,
wobei eine Kompatibilitätsprüfung (3012) eine aktuelle Software/Hardware (3008) des Steuermoduls (2100) mit der eingegebenen Software (3010) vergleicht, um zu bestimmen, ob die eingegebene Software (3010) kompatibel sein wird, und wenn das Ergebnis (3014) der Kompatibilitätsprüfung "Ja" ist, mindestens ein Modul (3020) umprogrammiert wird, zu dem die eingegebene Software (3010) gehört, und wenn das Ergebnis (3014) der Kompatibilitätsprüfung (3012) "Vielleicht" ist, zeigt das Steuermodul (2100) das Ergebnis (3014) der Kompatibilitätsprüfung (3012) an (3016), so dass der Benutzer einen Befehl (3018) zum Neuprogrammieren des Moduls (3020) trotz des "Vielleicht"-Ergebnisses (3014) initiieren kann.

2. Vorrichtung nach Anspruch 1, wobei das Steuermodul (2100) einen Upgrade-Anschluss (2105) zum Empfangen des Programms (1100) aufweist.

3. Vorrichtung mit einem der vorhergehenden Ansprüche, wobei das mindestens eine Eingangsmodul (2200; 2300; 2400) das Programm (1100) vom Steuermodul (2100) empfängt.

4. Vorrichtung mit einem der vorhergehenden Ansprüche, wobei das Umprogrammieren sowohl den Prozessor (2142; 2242; 2342; 2442) des mindestens einen Eingangsmoduls (2200; 2300; 2400) als auch den Prozessor (2142) des Steuermoduls (2100) neu konfiguriert.

5. Vorrichtung mit einem der vorhergehenden Ansprüche, die ferner ein Soft-Feature aufweist, das durch das Umprogrammieren aktiviert ist.

6. Vorrichtung nach einem der vorhergehenden Ansprüche, die ferner ein Soft-Feature aufweist, das durch das Umprogrammieren deaktiviert ist.

7. Vorrichtung mit einem der vorhergehenden Ansprüche, die ferner eine Modulverbindung (1000) aufweist, die das Steuermodul (2100) mit dem mindestens einen Eingangsmodul (2200; 2300; 2400) verbindet.

8. Vorrichtung mit einem der vorhergehenden Ansprüche, die ferner eine Netzwerkverbindung (7010) aufweist, wobei das Programm (1100) von der Netzwerkverbindung (7010) empfangen wird.

9. Vorrichtung nach Anspruch 8, wobei die Netzwerkverbindung (7010) drahtlos ist.

10. Vorrichtung mit einem der vorhergehenden Ansprüche, die ferner eine alternative Bildquelle (7206) aufweist, die mit dem Prozessor (2142) mit dem Umprogrammieren kompatibel ist.

11. Vorrichtung mit einem der vorhergehenden Ansprüche, die ferner eine Berechtigung zum Umprogrammieren (7014) aufweist, vom Prozessor (2142) empfangen wird.

12. Vorrichtung mit einem der vorhergehenden Ansprüche, die ferner ein Programm aufweist, das von dem Steuermodul (2100) empfangen wird, um einen Prozessor der Kamera (2206; 4000) neu zu programmieren.

## Revendications

1. Dispositif d'imagerie modulaire reprogrammable (2000) comprenant :
un module de commande (2100) ayant un processeur (2142) ;
au moins un module d'entrée (2200 ; 2300 ; 2400) ayant un processeur (2242 ; 2342 ; 2442) et raccordable audit module de commande (2100) ;
une caméra (2206 ; 4000) raccordable à l'au moins un module d'entrée (2200 ; 2300 ; 2400) destinée à transmettre des données d'image brutes (2204) à l'au moins un module d'entrée (2200 ; 2300 ; 2400) ;
ledit au moins un module d'entrée (2200 ; 2300 ; 2400) traitant lesdites données d'image brutes (2204) pour générer des données d'image traitées pour transmission audit module de commande (2100) ;
ledit module de commande (2100) recevant lesdites données d'image traitées et formatant lesdites données d'image traitées pour affichage ; et **caractérisé en ce que** ledit dispositif d'imagerie modulaire (2000) comprend en outre :
un programme (1100) reçu par ledit module de commande (2100) sous la forme d'un logiciel chargé (3010) pour reprogrammer à la fois le processeur (2242 ; 2342 ; 2442) dudit au moins un module d'entrée (2200 ; 2300 ; 2400) et le processeur (2142) dudit module de commande (2100),
dans lequel un contrôle de compatibilité (3012) compare un logiciel/matériel courant (3008) du module de commande (2100) au logiciel chargé (3010) pour déterminer si le logiciel chargé (3010) sera compatible, et si le résultat (3014) du contrôle de compatibilité est « oui », au moins un module (3020) auquel le logiciel chargé (3010) se rapporte est reprogrammé, et si le résultat (3014) du contrôle de compatibilité (3012) est « peut-être », le module de commande (2100) affiche (3016) le résultat (3014) du contrôle de compatibilité (3012) de telle sorte que l'utilisateur peut lancer une commande (3018) pour reprogrammer le module (3020) malgré le résultat « peut-être » (3014).

2. Dispositif de la revendication 1, dans lequel ledit module de commande (2100) a un port de mise à niveau (2105) destiné à recevoir le programme (1100).

3. Dispositif d'une quelconque revendication précédente, dans lequel l'au moins un module d'entrée (2200 ; 2300 ; 2400) reçoit le programme (1100) depuis le module de commande (2100).

4. Dispositif d'une quelconque revendication précédente, dans lequel ladite reprogrammation reconfigure à la fois le processeur (2242 ; 2342 ; 2442) dudit au moins un module d'entrée (2200 ; 2300 ; 2400) et le processeur (2142) dudit module de commande (2100).

5. Dispositif d'une quelconque revendication précédente comprenant en outre un élément logiciel activé par la reprogrammation.

6. Dispositif d'une quelconque revendication précédente comprenant en outre un élément logiciel désactivé par la reprogrammation.

7. Dispositif d'une quelconque revendication précédente comprenant en outre une liaison entre modules (1000) reliant le module de commande (2100) à l'au moins un module d'entrée (2200 ; 2300 ; 2400).

8. Dispositif d'une quelconque revendication précédente comprenant en outre une connexion réseau (7010), le programme (1100) reçu depuis ladite connexion réseau (7010).

9. Dispositif de la revendication 8 dans lequel la connexion réseau (7010) est sans fil.

10. Dispositif d'une quelconque revendication précédente comprenant en outre une source d'image alternative (7206) compatible avec le processeur (2142) lors de la reprogrammation.

11. Dispositif d'une quelconque revendication précédente comprenant en outre une autorisation de reprogrammation (7014) reçue par le processeur (2142).

12. Dispositif d'une quelconque revendication précédente comprenant en outre un programme reçu par le module de commande (2100) pour reprogrammer un processeur de la caméra (2206 ; 4000).
